(19) **Europäisches Patentamt**

**European Patent Office**

**Office européen des brevets**

(11) **EP 0 654 461 B1**

(12) **EUROPÄISCHE PATENTSCHRIFT**

(45) Veröffentlichungstag und Bekanntmachung des Hinweises auf die Patenterteilung:
**03.12.1997 Patentblatt 1997/49**

(51) Int. Cl.[6]: **C07C 68/00**, C07C 69/96

(21) Anmeldenummer: **94117665.3**

(22) Anmeldetag: **09.11.1994**

(54) **Verfahren zur Herstellung von Diarylcarbonaten**

Process for the preparation of diaryle carbonates

Procédé de préparation de carbonates de diaryle

(84) Benannte Vertragsstaaten:
**BE CH DE ES FR GB IT LI NL**

(30) Priorität: **22.11.1993 DE 4339697**
**09.12.1993 DE 4341990**

(43) Veröffentlichungstag der Anmeldung:
**24.05.1995 Patentblatt 1995/21**

(73) Patentinhaber: **BAYER AG**
**51368 Leverkusen (DE)**

(72) Erfinder:
- **Buysch, Hans-Josef**
  **D-47800 Krefeld (DE)**
- **Dohm, Joachim, Dr.**
  **D-51061 Köln (DE)**
- **Hesse, Carsten, Dr.**
  **D-47800 Krefeld (DE)**
- **Rechner, Johann, Dr.**
  **D-47800 Krefeld (DE)**
- **Kaufmann, Dieter, Prof. Dr.**
  **D-38640 Goslar (DE)**

(56) Entgegenhaltungen:
**EP-A- 0 350 700          DE-A- 2 815 512**

## Beschreibung

Die vorliegende Erfindung betrifft ein Verfahren zur Herstellung von Diarylcarbonaten durch Umsetzung einer aromatischen Hydroxyverbindung (z.B. Phenol) mit Kohlenmonoxid und Sauerstoff bei erhöhter Temperatur in Gegenwart einer Base, eines quartären Salzes, eines Trockenmittels, eines Katalysators und eines Cokatalysators, das dadurch gekennzeichnet ist, daß man den Katalysator vor der Reaktion aktiviert. In einer besonderen Verfahrensvariante wird als Base ein vorgeformtes Alkalimetallphenolat eingesetzt.

Es ist bekannt, aromatische Carbonate durch oxidative Umsetzung einer aromatischen Hydroxyverbindung mit Kohlenmonoxid in Gegenwart eines homogenen Edelmetall-Katalysators herzustellen (DE-OS 28 15 512). Als Edelmetalle werden die Elemente der Gruppe VIIIb vorgeschlagen, wobei bevorzugt Palladium eingesetzt wird. Während der Reaktion wird diese Palladium(II)-Spezies zu Palladium(0) reduziert und durch Sauerstoff mit Hilfe eines Cokatalysators wieder zu Palladium(II) oxidiert. Als Cokatalysatoren können z.B. verschiedene Mangan-oder Kobaltsalze in unterschiedlichen Oxidationsstufen eingesetzt werden. Neben diesen Cokatalysatoren werden eine Base, ein Phasen-Transfer-Katalysator und ein Trockenmittel eingesetzt. Als Lösungsmittel wird bevorzugt Methylenchlorid verwendet. Gemäß DE-OS 27 38 437 werden sterisch gehinderte tertiäre Amine als Base und ein Molsieb als Trockenmittel eingesetzt.

Nachteile dieser Verfahren sind neben dem Einsatz des toxischen, leichtflüchtigen Methylenchlorids als Lösungsmittel, das einen hohen sicherheitstechnischen Aufwand erfordert und kostenintensiv zurückgewonnen werden muß, lange Reaktionszeiten und die damit verbundenen schlechten Raum-Zeit-Ausbeuten. Für eine technische Umsetzung erweist sich jedoch die ungenügende Reproduzierbarkeit als der eigentlich entscheidende Nachteil, da man von Ansatz zu Ansatz bei gleicher Verfahrensweise ganz unterschiedliche Resultate, ja sogar völliges Versagen der Katalyse erhalten kann.

Der hohe Preis und die oxidative Unbeständigkeit der in DE-OS 27 38 437 vorgeschlagenen sterisch gehinderten tertiären Amin-Basen ist ein weiterer Nachteil dieses Verfahrens. Für die Wiedergewinnung der Base muß ein hoher technischer Aufwand betrieben werden. Außerdem wird ein erheblicher Teil der Base im Laufe der langen Reaktionszeiten zersetzt, so daß ständig große Mengen der teuren Base ersetzt werden müssen, was eine wirtschaftliche Nutzung des Verfahrens erschwert.

J.E. Hallgren und G.M. Lucas berichten im Journal of Organometallic Chemistry 212 (1981) 135-139 über den Einsatz von wäßriger Natriumhydroxid-Lösung als Base. In Gegenwart kleiner Mengen 50%iger wäßriger Natriumhydroxid-Lösung und eines Phasen-Transfer-Katalysators beobachten Hallgren und Lucas eine Erhöhung der Reaktionsgeschwindigkeit gegenüber dem Einsatz tertiärer Amine. Einen erheblichen Nachteil dieser Vorgehensweise stellt die Tatsache dar, daß aromatische Carbonate, die durch dieses Verfahren hergestellt werden sollen, durch wäßrige Natriumhydroxid-Lösung schnell zersetzt werden (Ullmanns Encyclopädie, 5. Aufl., Vol. A5, S. 197-202). Die Spaltungsreaktion aromatischer Carbonate läuft auch in Gegenwart katalytischer Mengen wäßriger Natriumhydroxid-Lösung so schnell ab, daß im Reaktionssystem nur geringe Carbonat-Konzentrationen erzielt werden können. Außerdem wird auch in diesem Fall das toxische, leichtflüchtige Methylenchlorid als Lösungsmittel eingesetzt, das die oben beschriebenen Probleme mit sich bringt. Zusätzlich geht von der gleichzeitigen Anwesenheit von Methylenchlorid und Natriumhydroxid eine besondere Gefahr aus, da diese sich, wie dem Fachmann bekannt ist, zu hochreaktivem Dichlorcarben umsetzen, das zu Nebenreaktionen führt, gegebenenfalls sogar spontan und explosionsartig reagieren kann. Wegen dieser unkontrollierbaren Reaktion ist eine technische Umsetzung auch dieses Verfahrens nicht möglich. Darüber hinaus verbessern diese Maßnahmen die Reproduzierbarkeit nicht.

In der EP-503 581 wird die Verwendung von verschiedenen Kupfersalzen als Cokatalysator vorgeschlagen. Neben diesem Cokatalysator wird auch die Verwendung erheblicher Mengen verschiedener Chinone/Hydrochinone als Elektronen-Transfer-Katalysator vorgeschlagen. Diese Maßnahmen verbessern die Reproduzierbarkeit nicht. Damit ist die technische Umsetzung auch dieses Verfahrens nicht möglich. Außerdem wird in diesem Verfahren als Lösungsmittel ebenfalls Methylenchlorid eingesetzt. Die Abtrennung des Elektronen-Transfer-Katalysators aus dem Reaktionsgemisch erfordert in diesem Verfahren einen zusätzlichen erheblichen Aufwand. Zudem stellen Hydrochinone eine aromatische bifunktionelle Hydroxyverbindung dar, die in gleicher Weise wie Phenol zu Carbonaten umgesetzt werden kann. Die Abtrennung der auf diese Weise gebildeten Nebenprodukte kann nur mit großem Aufwand erreicht werden. Eine Wiedergewinnung des eingesetzten Elektronen-Transfer-Katalysators ist somit nicht möglich. Die Bildung der Nebenprodukte würde bei gegebener Reproduzierbarkeit die Selektivität und damit die Wirtschaftlichkeit dieses Prozesses erheblich senken.

In der Anmeldung WO 93/03000 wird ein Verfahren zur Herstellung aromatischer Carbonate beschrieben, das zwar auf die Verwendung eines Lösungsmittels verzichtet, aber weiterhin erhebliche Mengen an Cokatalysator und Elektronen-Transfer-Katalysator benötigt. Auch dadurch wird das Problem der ungenügenden Reproduzierbarkeit nicht gelöst, so daß insgesamt ein technisch umsetzbares Verfahren bisher nicht zur Verfügung steht.

Es bestand daher die Aufgabe, ein Verfahren zu finden, das es erlaubt, die Synthese aromatischer Carbonate unter technisch realisierbaren Bedingungen reproduzierbar durchzuführen.

Überraschenderweise wurde nun gefunden, daß man die Herstellung von Diarylcarbonaten durch oxidative Carbonylierung einer aromatischen Hydroxyverbindung in Gegenwart eines Edelmetall-Katalysators, eines Cokatalysators, eines Trockenmittels, eines quartären Salzes und einer Base ohne Zusatz eines Elektronen-Transfer-Katalysators reproduzierbar durchführen kann, wenn man den Edelmetall-Katalysator vor der Reaktion in Gegenwart des quartären Salzes in flüssiger Phase, also einem Lösungsmittel oder der aromatischen Hydroxyverbindung allein oder in einer Mischung aus Lösungsmittel und aromatischer Hydroxyverbindung, mit Kohlenmonoxid aktiviert. Diese Aktivierung des Edelmetall-Katalysators führt nicht nur zu einer guten Reproduzierbarkeit der Umsetzungen, sondern auch überraschenderweise zur Erhöhung der Raum-Zeit-Ausbeuten.

Weiterhin wurde gefunden, daß die oben beschriebene Aktivierung auch in Anwesenheit des hierzu nicht erforderlichen Cokatalysators ohne Einschränkung gelingt.

Aktiviert man den Edelmetallkatalysator zusätzlich in Gegenwart des hierzu nicht erforderlichen Cokatalysators, hat dies den Vorteil, daß nach erfolgter Aufarbeitung Edelmetall- und Cokatalysator gemeinsam rezyklisiert werden können und auf aufwendige Trennoperationen verzichtet werden kann.

Die Erfindung betrifft demnach ein Verfahren zur Herstellung eines organischen Carbonats der Formel (I)

$$R-O-CO-O-R \qquad (I),$$

in der

R ein $C_6$-$C_{12}$-Aryl, das ein- bis zweimal durch $C_1$-$C_4$-Alkyl, $C_1$-$C_4$-Alkoxy, Fluor, Chlor oder Brom substituiert sein kann, bedeutet,

durch Umsetzung einer aromatischen Hydroxyverbindung der Formel (II),

$$R-O-H \qquad (II),$$

worin

R die oben angegebene Bedeutung hat,

mit Kohlenmonoxid und Sauerstoff bei 30-200°C, bevorzugt 30-150°C, besonders bevorzugt 40-120°C und bei einem Druck von 1-80 bar, bevorzugt 2-50 bar, besonders bevorzugt 5-25 bar in Gegenwart einer Verbindung eines Edelmetalls der Gruppe VIIIb als Katalysator, eines Cokatalysators aus der Gruppe der Metallverbindungen der Gruppen IIIA, IVA, VA, IB, IIB, VIB und VIIB des Periodensystems der Elemente (Mendelejew), eines quartären Salzes, eines Trockenmittels und einer Base, das dadurch gekennzeichnet ist, daß man den Edelmetallkatalysator vor der Reaktion in Gegenwart des quartären Salzes und in Gegenwart oder in Abwesenheit des Cokatalysators durch Behandlung mit Kohlenmonoxid in flüssiger Phase bei einer Temperatur von 15-200°C, bevorzugt 20-150°C, besonders bevorzugt 40-100°C und einem Druck von 1-300 bar, bevorzugt 1-200 bar, besonders bevorzugt 1-150 bar, aktiviert, wobei der Edelmetall-Katalysator im Aktivierungsansatz in einer Menge von 0,0001-30 Gew.-%, bezogen auf das gesamte Reaktionsgemisch, vorliegt.

Am Beispiel der Bildung von Diphenylcarbonat (DPC) kann das erfindungsgemäße Verfahren wie folgt formelmäßig dargestellt werden:

$$2\ C_6H_5 - OH + CO + 1/2\ O_2 \rightarrow (C_6H_5\ O)_2CO + H_2O.$$

Zur Aktivierung wird der Edelmetall-Katalysator, dessen Menge im erfindungsgemäßen Verfahren nicht beschränkt ist, bevorzugt aber so bemessen wird, daß die Konzentration des Metalls im Aktivierungsansatz 0,0001 bis 30 Gew.-%, besonders bevorzugt 0,001 bis 10 Gew.-%, beträgt, in einem inerten Lösungsmittel oder direkt in der Schmelze der aromatischen Hydroxyverbindung oder in Mischungen beider gelöst.

Der Cokatalysator, dessen Menge bei der erfindungsgemäßen Aktivierung nicht beschränkt ist, bevorzugt aber so bemessen wird, daß die Konzentration im Aktivierungsansatz im Bereich von 0 bis 40 Gew.-% liegt, bevorzugt ist eine Menge von 0,005 bis 10 Gew.%, besonders bevorzugt 0,01 bis 4 Gew.%, bezogen auf den gesamten Aktivierungsansatz.

Der Cokatalysator ist für die Aktivierung des Edelmetallkatalysators nicht erforderlich, stört diese jedoch auch nicht. Die Cokatalysatorzugabe zu diesem Zeitpunkt erleichtert eine kontinuierliche Betriebsweise, da erstens eine eigene Cokatalysatordosierung entfällt und zweitens Edelmetall und Cokatalysator bei der Aufarbeitung der Reaktionstörungen nicht getrennt werden müssen.

Als für die erfindungsgemäße Aktivierung geeignete Lösungsmittel seien aliphatische Kohlenwasserstoffe, cycloaliphatische Kohlenwasserstoffe, aromatische Kohlenwasserstoffe, halogenierte Kohlenwasserstoffe, wie beispielsweise Methylenchlorid, Ether und Ester genannt.

Zu dieser Lösung wird ein quartäres Salz gegeben, bei dem es sich beispielsweise um mit organischen Resten substituierte Ammonium- oder Phosphoniumsalze handelt. Geeignet für den Einsatz im erfindungsgemäßen Verfahren sind Ammonium- und Phosphoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid, Tetrafluoroborat oder Hexafluorophosphat tragen. Bevorzugt werden im erfindungsgemäßen Verfahren Ammoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, eingesetzt, besonders bevorzugt ist Tetrabutylammoniumbromid.

Diese Lösung wird anschließend bei 15 bis 200°C, bevorzugt bei 20 bis 150°C, besonders bevorzugt bei 40 bis 100°C, mit Kohlenmonoxid behandelt. Das kann sowohl dadurch geschehen, daß man bei Normaldruck pro Gramm des eingesetzten Edelmetalls Kohlenmonoxid in einer Menge von 0,1 bis 250 l/h, bevorzugt 0,5 bis 200 l/h, besonders bevorzugt 1 bis 100 l/h einleitet, als auch dadurch, daß man die Lösung in einem Autoklaven unter einem Druck von 1 bis 300 bar, bevorzugt 1 bis 200 bar, besonders bevorzugt 1 bis 150 bar mit Kohlenmonoxid versetzt. Die Aktivierungszeit hängt geringfügig vom verwendeten Edelmetallkatalysator und von einem gegebenenfalls eingesetzten inerten Lösungsmittel ab. Sie beträgt im allgemeinen wenige Minuten bis einige Stunden, beispielsweise 0,05-5 h, bevorzugt 0,1-3 h, besonders bevorzugt 0,25-2 h. Im erfindungsgemäßen Verfahren kann der Edelmetall-Katalysator direkt vor der Reaktion aktiviert werden, er kann aber auch nach Abtrennung des Lösungsmittels oder der aromatischen Hydroxyverbindung, z.B. durch Abdestillieren, isoliert und gelagert werden. Dieser einmal aktivierte Edelmetall-Katalysator bleibt auch nach Monaten an der Luft stabil und kann ohne Aktivitätsverlust eingesetzt werden. Auch eine Lagerung in gelöster Form ist möglich. Eine weitere Ausführungsform des erfindungsgemäßen Verfahrens kann darin bestehen, daß man den Edelmetall-Katalysator in der oben beschriebenen Weise aktiviert und diese Lösung dann einmalig oder in mehreren Portionen dem Reaktionssystem zusetzt.

Bei den in das erfindungsgemäße Verfahren einzusetzenden aromatischen Hydroxyverbindungen handelt es sich um aromatische Hydroxyverbindungen, die von $C_6$-$C_{12}$-Aromaten, wie Benzol, Naphthalin oder Biphenyl, abgeleitet sind und ein- bis zweimal durch $C_1$-$C_4$-Alkyl, $C_1$-$C_4$-Alkoxy, Fluor, Chlor oder Brom substituiert sein können, bevorzugt Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol, besonders bevorzugt um Phenol.

Das erfindungsgemäße Verfahren wird vorzugsweise ohne Lösungsmittel durchgeführt. Selbstverständlich können auch inerte Lösungsmittel verwendet werden. Als solche seien die gleichen genannt wie die oben für die Aktivierung erwähnten.

Die für das erfindungsgemäße Verfahren geeigneten Edelmetall-Katalysatoren bestehen aus mindestens einem Metall der Gruppe VIII, vorzugsweise Palladium. Es kann bei dem erfindungsgemäßen Verfahren in verschiedener Form zugegeben werden. Palladium kann in metallischer Form oder bevorzugt in Form von Palladium-Verbindungen der Oxidationsstufen 0 und +2, wie beispielsweise Palladium(II)-acetylacetonat, -halogeniden -carboxylaten von $C_2$-$C_6$-Carbonsäuren, -nitrat, -oxiden oder Palladiumkomplexen, die beispielsweise Olefine, Amine, Phosphine und Halogenide enthalten können, eingesetzt werden. Besonders bevorzugt ist Palladiumbromid, Palladium(II)-acetylacetonat und Palladiumacetat.

Die Menge an Edelmetall-Katalysator ist im erfindungsgemäßen Verfahren nicht beschränkt. Bevorzugt wird so viel Katalysator zugesetzt, daß seine Konzentration, gerechnet als Metall, im Reaktionsansatz 10 bis 3000 ppm beträgt, besonders bevorzugt sind Konzentrationen von 50 bis 1000 ppm.

Als Cokatalysator für das erfindungsgemäße Verfahren wird eine Metallverbindung der Gruppen III A, IV A, V A, I B, II B, VI B oder VII B des Periodensystems der Elemente (Mendelejew) eingesetzt, wobei das Metall in verschiedenen Oxidationsstufen eingesetzt werden kann. Ohne das erfindungsgemäße Verfahren einzuschränken, seien Mangan(II), Mangen(III), Kupfer(I), Kupfer(II), Kobalt(II), Kobalt(III), Vanadium(III) und Vanadium(IV) genannt. Die Metalle können beispielsweise als Halogenide, Oxide, Carboxylate von $C_2$-$C_6$-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Bevorzugt werden Mangen(II)-Verbindungen im erfindungsgemäßen Verfahren verwendet, besonders bevorzugt Mangan(II)-Komplexe, ganz besonders bevorzugt Mangan(II)acetylacetonat.

Der Cokatalysator wird der Reaktionslösung in einer Menge zugesetzt, daß seine Konzentration im Bereich von 0,001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%. Der Cokatalysator kann gemeinsam mit dem aktivierten Edelmetallkatalysator oder weniger bevorzugt einzeln zugesetzt werden.

Trockenmittel für das erfindungsgemäße Verfahren sind vorzugsweise inert und von der dem Fachmann bekannten Art zur Bindung von Wasser. Sie können in regenerative und nicht regenerative, flüssige oder feste, in chemisch reaktive d.h. solche, die eine neue Verbindung oder ein Hydrat bilden, in physikalisch absorptive mit konstanter oder variabler relativer Feuchtigkeit in Adsorptionsmittel usw. klassifiziert werden. Vorzugsweise haben das oder die im erfindungsgemäßen Verfahren verwendete(n) Trockenmittel eine hohe Kapazität und/oder Wirksamkeit, vorzugsweise beides, bei der Entfernung von Feuchtigkeit aus dem Reaktionsmedium. Der in der vorliegenden Anmeldung verwendete Begriff "Kapazität" bezieht sich auf die Menge Wasser, die von einer gegebenen Gewichtsmenge des Trocknungsmittels aufgenommen werden kann, und der Begriff "Wirksamkeit" bezieht sich auf den Trocknungsgrad, der durch ein solchen Trockenmittel erreicht werden kann. Als Beispiele für derartige Trockenmittel seien, ohne das erfindungsgemäße Verfahren auf diese Beispiele einzuschränken, aktiviertes Aluminiumoxid, Bariumoxid, Calciumoxid, Calciumchlorid, Calciumsulfat, Lithiumchlorid, Natriumsulfat, Magnesiumsulfat und natürliche oder synthetische hydrophile Alumosilikate des Zeolith-Typs (Molekularsiebe) genannt. Bevorzugte Trocknungsmittel für die Verwendung im erfindungsgemäßen Verfahren sind natürliche oder synthetische hydrophile Alumosilikate des Zeolith-Typs (Molekularsiebe). Besonders bevorzugt werden Zeolithe von Typ A oder Faujasit eingesetzt.

In das erfindungsgemäße Verfahren wird diejenige Menge Trockenmittel eingesetzt, die zur Aufnahme des gebildeten Reaktionswasser und der Feuchtigkeit der Edukte ausreicht. Diese Menge hängt von Kapazität und Wirksamkeit des jeweils eingesetzten Trockenmittels ab und kann vom Fachmann jeweils errechnet werden. So ist beispielsweise bekannt, daß bei Zeolith A mit einer Wasseraufnahme von 20 - 30 % seines Trockengewichts gerechnet werden kann. Man wird erfindungsgemäß mit einer Menge Trockenmittel arbeiten, die 100 - 800 %, bevorzugt 200 - 600 %, der erwarteten und aufzunehmenden Wassermenge entspricht. Setzt man weniger als diese Menge Trockenmittel ein, werden schlechtere Ergebnisse erhalten; setzt man mehr Trockenmittel ein, als die Obergrenze angibt, ist die Wirtschaftlichkeit des Verfahrens in Frage gestellt, da viel Trockenmittel ohne Ausnutzung im Kreis gefahren wird. Die Obergrenze, die absolute Menge an Trockenmittel und damit auch der zu erwartende Umsatz zu Carbonaten (I) sind auch dadurch begrenzt, daß das Reaktionsgemisch noch ruhrbar oder anderweitig mischbar bleibt. Im allgemeinen wird eine Menge von 1 bis 30 Gew.-%, bezogen auf das Reaktionsgemisch, eingesetzt.

Das quartäre Salz im erfindungsgemäßen Verfahren hat den oben für die Katalysatoraktivierung genannten Umfang. Es kann für Aktivierung und Verfahrensdurchführung gleich oder verschieden, im Sinne einer Vereinfachung der Aufarbeitung jedoch bevorzugt gleich sein.

Die Menge eines solchen quartären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Für das erfindungsgemäße Verfahren können tertiäre Amine oder Alkalimetallhydroxide bzw. -carbonate verwendet werden. Als tertiäre Amine kommen solche in Frage, die als organische Reste $C_6$- bis $C_{10}$-Aryl-, $C_7$- bis $C_{12}$-Aralkyl- und/oder $C_1$- bis $C_{20}$-Alkyl-Reste tragen, beispielsweise Triethylamin, Tripropylamin, Tributylamin, Trioctylamin, Benzyldimethylamin, Dioctylbenzylamin, Dimethylphenethylamin, 1-Dimethylamino-2-phenylpropan; tertiäre Amine für das erfindungsgemäße Verfahren sind weiterhin cyclische aromatische oder nicht aromatische, wie Pyridin, N-Methylpiperidin, 1,2,2,6,6-Pentamethylpiperidin. Besonders bevorzugt sind sterisch gehinderte tertiäre Amine, z.B. Diisopropylethylamin oder 1,2,2,6,6-Pentamethylpiperidin.

Die Base wird in einer von der Stöchiometrie unabhängigen Menge zugesetzt. Das Verhältnis von Palladium zu Base wird vorzugsweise so gewählt, daß pro Mol Palladium 0,1 bis 5, bevorzugt 0,5 bis 2, besonders bevorzugt 0,9 bis 1,3 Äquivalente Base bezogen auf Palladium eingesetzt werden.

In einer besonderen Verfahrensvariante wird als Base mit Vorteil ein vorgeformtes Alkalimetallphenolat oder eine Lösung eines vorgeformten Alkalimetallphenolats eingesetzt. Dies ist sehr überraschend, als der Einsatz von Alkalimetallhydroxiden oder tertiären Aminen im Reaktionsgemisch zur Bildung von Phenolaten führen sollte. Setzt man jedoch vorgeformte Alkalimetallphenolate als Base unter sonst gleichen Bedingungen ein, werden deutliche höhere Selektivitäten und Raum-Zeit-Ausbeuten erzielt als bei der Verwendung von tertiären Aminen oder Alkalimetallhydroxiden. Eine Erklärung für dieses Phänomen gibt es derzeit nicht. Es wird vermutet, daß die in Gegenwart basischer Verbindungen erfolgende Ausfällung von Katalysator und Cokatalysator durch Phenolatanionen anders erfolgt als durch tertiäre Amine, Alkalimetallhydroxide oder -carbonate. Die durch Phenolatanionen erfolgte Ausfällung entfaltet offenbar eine höhere Aktivität. In Systemen, in denen aus aromatischer Hydroxyverbindung und tert.-Aminen oder Alkalimetallhydroxiden oder -carbonaten Phenolatanionen gebildet werden können, erfolgt offenbar die Bildung einer weniger aktiven Fällung so rasch, daß etwa gebildetes Phenolat diese Tatsache nicht mehr korrigieren kann. Dieser Befund ist höchst überraschend.

In das erfindungsgemäße Verfahren einsetzbare Alkalimetallphenolate stellen Alkalisalze von aromatischen Hydroxyverbindungen der Formel (II) dar, in der R die oben angegebene Bedeutung hat. Ganz besonders bevorzugt wird ein Alkalisalz der aromatischen Hydroxyverbindung verwendet, die auch zu organischem Carbonat umgesetzt werden soll.

Als Kationen für die erfindungsgemäßen Alkalimetallphenolate sind die Alkalimetalle Lithium, Natrium, Kalium, Rubidium oder Cäsium geeignet. Bevorzugt werden Lithium-, Natrium- und Kaliumphenolate, besonders bevorzugt Natrium- und Kaliumphenolat eingesetzt.

Das Alkalimetallphenolat kann dem Reaktionsgemisch als reine Verbindung in fester Form oder als Schmelze zugesetzt werden. Selbstverständlich können in das erfindungsgemäße Verfahren auch die Hydrate der Alkalimetallphenolate eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist allgemein so bemessen, daß pro Mol Phenolat maximal 5 Mol Wasser eingesetzt werden. Höhere Wasserkonzentrationen führen i.a. zu schlechteren Umsätzen und Zersetzung gebildeter Carbonate, wie im Fall der von Hallgren vorgeschlagenen wäßrigen Natriumhydroxid-Lösung. In bevorzugter Weise werden maximal 2 Mol Wasser, besonders maximal 1 Mol Wasser pro Mol Phenolat eingesetzt. In besonders bevorzugter Weise werden im wesentlichen wasserfreie Phenolate eingesetzt, wie sie beispielsweise technisch verfügbar sind.

In einer weiteren Ausführungsform der Erfindung wird das Alkalimetallphenolat dem Reaktionsgemisch als Lösung, die 0,1 bis 80 Gew.-%, bevorzugt 0,5 bis 65 Gew.-%, besonders bevorzugt 1 bis 50 Gew.-% des Alkalimetallphenolats enthält, zugesetzt. Als Lösungsmittel können hierbei sowohl $C_1$-$C_8$-Alkohole oder Phenole (II), als auch weitere Lösungsmittel verwendet werden. Als Beispiele für weitere Lösungsmittel seien Dimethylacetamid, N-Methyl-pyrrolidinon, Tetramethylharnstoff, Etheralkohole, wie Diethylenglykol, halogenierte Kohlenwasserstoffe, z.B. Chlorbenzol oder Dichlorbenzol, und Ether, wie Dioxan, genannt. Diese Lösungsmittel können allein oder in beliebiger Kombination miteinander eingesetzt werden. So besteht eine Ausführungsform des erfindungsgemäßen Verfahrens beispielsweise darin, daß man das Phenolat in einer Phenolschmelze löst, die mit einem inerten Lösungsmittel verdünnt wurde. Bevorzugt wird das Alkalimetallphenolat in der Schmelze einer aromatischen Hydroxyverbindung gelöst. Besonders bevorzugt wird das Alkalimetallphenolat in einer Schmelze der aromatischen Hydroxyverbindung gelöst, die zum organischen Carbonat umgesetzt werden soll. Ganz besonders bevorzugt wird das Alkalimetallphenolat in Phenol gelöst.

Zu solchen Lösungen kann man z.B. kommen, indem man das Alkalimetallphenolat direkt mit dem Lösungsmittel zusammengibt. Natürlich kann man auch Alkalimetalle, Alkalimetall(hydro)oxide oder -alkoholate mit Phenol, gegebenenfalls in Gegenwart eines zusätzlichen inerten Lösungsmittel, zum Alkalimetallphenolat umsetzen, das dabei gebildete Wasser bzw. den gebildeten Alkohol z.B. durch Destillieren entfernen und auf diese Weise zu einer in das erfindungsgemäße Verfahren einsetzbaren Lösung eines Alkalimetallphenolats kommen.

Die Phenolatmenge ist nicht kritisch. Das Verhältnis von Edelmetall zu Phenolat wird vorzugsweise so gewählt, daß pro Mol Edelmetall 0,1 bis 500, bevorzugt 0,5 bis 200, besonders bevorzugt 0,9 bis 130 Äquivalente Phenolat eingesetzt werden.

Das erfindungsgemäße Verfahren wird, vorzugsweise ohne Lösungsmittel, bei 30 bis 200°C, bevorzugt bei 30 bis 150°C, besonders bevorzugt bei 40 bis 120°C bei einem Druck von 1 bis 80 bar, bevorzugt von 2 bis 50 bar, besonders bevorzugt von 5 bis 25 bar durchgeführt.

Die Zusammensetzung der Reaktionsgase Kohlenmonoxid und Sauerstoff kann in weiten Konzentrationsgrenzen variiert werden, es wird jedoch zweckmäßigerweise ein $CO:O_2$-Molverhältnis (normiert auf CO) von 1:(0,001-1,0) bevorzugt 1:(0,01 - 0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen, so kann Synthesegas als CO-Quelle und Luft als $O_2$-Träger dienen; es ist lediglich darauf zu achten, daß keine Katalysatorgifte wie z.B.: Schwefel oder dessen Verbindungen eingetragen werden. In der bevorzugten Ausführungsform des erfindungsgemäßen Verfahrens werden reines CO und reiner Sauerstoff verwendet.

Das erfindungsgemäße Verfahren kann in verschiedenen Ausführungsvarianten ausgeübt werden. Eine Möglichkeit besteht in der diskontinuierlichen Durchführung in üblichen Rührgefäßen oder Autoklaven. Dabei werden CO und Sauerstoff entweder durch einen Begasungsrührer oder andere bekannte Gasverteilungsorgane in die Reaktionsmischung geleitet. Nach Erreichen des optimalen Umsatzes wird das Reaktionsgemisch z.B. durch Destillation aufgearbeitet, wobei zunächst die aromatische Hydroxyverbindung abgetrennt und in einem weiteren Schritt das aromatische Carbonat isoliert wird. Die im Rückstand befindlichen Katalysatorkomponenten und das Trockenmittel können nach üblichen Maßnahmen zurückgewonnen und recyclisiert werden.

Die folgenden Beispiele verdeutlichen das erfindungsgemäße Verfahren, ohne es jedoch auf diese einzuschränken.

## Beispiel 1:

Katalysatoraktivierung:

In einem Reaktor wurden 0,133 g Palladiumbromid und 3,2 g Tetrabutylammoniumbromid bei 55°C in 100 g Phenol mit 750 ppm $H_2O$ ($\triangleq$ 4 mmol $H_2O$/100 g) gelöst. Durch einen Begasungsrührer wurde eine Stunde Kohlenmonoxid (3 l/h) eingeleitet. Dabei färbte sich das Reaktionsgemisch orange.

Reaktionsdurchführung:

Zu der Lösung des aktivierten Katalysators wurden 4 g Zeolith A (Baylith L 133 der Fa. Bayer AG), 0,153 g Mangan(II)acetylacetonat und 0,403 g 1,2,2,6,6-Pentamethylpiperidin gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 1,5 % Diphenylcarbonat im Reaktionsgemisch enthalten waren.

Bei der Annahme einer Bindungsfähigkeit von Zeolith A für $H_2O$ von 20 % seines Gewichts können 4 g Zeolith A 0,8 g = 44 mmol $H_2O$ binden. Das ergibt, abzüglich 4 mmol $H_2O$ im eingesetzten Phenol, eine Kapazität von 40 mmol für Reaktionswasser. 1,5 % Diphenylcarbonat entsprechen 1,62 g bzw. 7,57 mmol. Die 7,57 mmol Reaktionswasser lasteten den Zeolith A zu etwa 19 % seiner Kapatität von 40 mmol $H_2O$ aus. Der Zeolith A lag somit in einer Menge von etwa 530 % der benötigten Menge vor.

Dieser Versuch wurde, wie beschrieben, wiederholt. Die Analyse ergab, daß 1,55 % Diphenylcarbonat im Reaktionsgemisch enthalten waren. Eine weitere Wiederholung ergab erneut 1,5 % Diphenylcarbonat im Reaktionsgemisch.

**Vergleichsbeispiel 1:**

In einem Reaktor wurden 0,133 g Palladiumbromid, 3,2 g Tetrabutylammoniumbromid, 4 g Zeolith A (Baylith L 133 der Fa. Bayer AG), 0,153 g Mangan(II)-acetylacetonat und 0,403 g 1,2,2,6,6-Pentamethylpiperidin bei 55°C in 100 g Phenol gelöst. Dann wurde wie in Beispiel 1 beschrieben ein Gasgemisch aus Kohlenmonoxid und Luft für 6 Stunden eingeleitet und das Reaktionsgemisch anschließend gaschromatographisch analysiert. Die Analyse ergab, daß 0,25 % Diphenylcarbonat im Reaktionsgemisch enthalten waren.

Dieser Versuch wurde mit frischem Katalysator in gleicher Weise wiederholt. Die Analyse ergab, daß kein Diphenylcarbonat im Reaktionsgemisch enthalten war.

Danach wurde dieser Versuch erneut und wiederum mit frischem Katalysator wie beschrieben wiederholt. Die Analyse nach 6 Stunden Reaktionszeit ergab, daß 0,8 % Diphenylcarbonat im Reaktionsgemisch enthalten waren.

Diese Beispiele zeigen, daß keine reproduzierbaren Ergebnisse zu erzielen sind.

**Beispiel 2:**

Die Katalysatoraktivierung erfolgte wie in Beispiel 1 beschrieben.
Zu der Lösung des aktivierten Katalysators wurden 4 g Zeolith A (Baylith L 133 der Fa. Bayer AG), 0,153 g Mangan(II)acetylacetonat und 0,4 ml 50 %ige wäßrige Natronlauge gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 1,4 % Diphenylcarbonat im Reaktionsgemisch enthalten waren.

Der $H_2O$-Anteil der Natronlauge entsprach 11 mmol. Die verbleibende $H_2O$-Bindungskapazität des Zeolith A betrug somit 29 mmol. 1,4 % Diphenylcarbonat entsprechen 6,5 mmol. Somit wurde die $H_2O$-Bindungskapazität zu 22 % ausgelastet. Der Zeolith A lag damit in einer Menge von 455 % der benötigten Menge vor.

Dieser Versuch wurde wie beschrieben wiederholt. Die Analyse ergab, daß 1,36 % Diphenylcarbonat im Reaktionsgemisch enthalten waren.

**Beispiel 3:**

Katalysatoraktivierung:

In einem Reaktor wurden 0,133 g Palladiumbromid, 0,153 g Mangan(II)acetylacetonat und 3,2 g Tetrabutylammoniumbromid bei 55°C in 100 g Phenol mit 750 ppm $H_2O$ ($\triangle$ 4 mmol $H_2O$/100 g) gelöst. Durch einen Begasungsrührer wurde eine Stunde Kohlenmonoxid (3 l/h) eingeleitet.

Reaktionsdurchführung:

Zu der Lösung des aktivierten Katalysators wurden 4 g Zeolith A (Baylith L 133 der Fa. Bayer AG) und 0,4 ml 25%ige wäßrige Natronlauge gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 1,4% Diphenylcarbonat im Reaktionsgemisch enthalten waren.

Dieser Versuch wurde, wie beschrieben, wiederholt. Die Analyse ergab, daß 1,5% Diphenylcarbonat im Reaktionsgemisch enthalten waren.

**Beispiel 4:**

Katalysatoraktivierung:

In einem Reaktor wurden 0,152 g Palladiumacetylacetonat, 0,153 g Mangan(II)-acetylacetonat und 3,2 g Tetrabutylammoniumbromid bei 55°C in 100 g Phenol mit 750 ppm $H_2O$ ($\triangle$ 4 mmol $H_2O$/100 g) gelöst. Durch einen Begasungsrührer wurde eine Stunde Kohlenmonoxid (3 l/h) eingeleitet.

Reaktionsdurchführung:

Zu der Lösung des aktivierten Katalysators wurden 4 g Zeolith A (Baylith L 133 der Fa. Bayer AG) und 0,4 ml 25%ige wäßrige Natronlauge gegeben. Dann wurde ein Gasgemisch (12 l/h) aus Kohlenmonoxid und Luft (1:1) für 6 Stunden eingeund das Reaktionsgemisch anschließend gaschromatographisch analysiert. Die Analyse ergab, daß 1,55% Diphenylcarbonat im Reaktionsgemisch enthalten waren.

Dieser Versuch wurde, wie beschrieben, wiederholt. Die Analyse ergab, daß 1,45% Diphenylcarbonat im Reaktionsgemisch enthalten waren.

**Beispiel 5**

Katalysatoraktivierung:

In einem Reaktor wurden 0,112 g Palladiumacetat, 0,153 g Mangan(II)acetylacetonat und 3,2 g Tetrabutylammoniumbromid bei 55°C in 100 g Phenol mit 750 ppm $H_2O$ ($\triangle$ 4 mmol $H_2O$/100 g) gelöst. Durch einen Begasungsrührer wurde eine Stunde Kohlenmonoxid (3 l/h) eingeleitet.

Reaktionsdurchführung:

Zu der Lösung des aktivierten Katalysators wurden 4 g Zeolith A (Baylith L 133 der Fa. Bayer AG) und 0,4 ml 25%ige wäßrige Natronlauge gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 1,2% Diphenylcarbonat im Reaktionsgemisch enthalten waren.

Dieser Versuch wurde, wie beschrieben, wiederholt. Die Analyse ergab, daß 1,25% Diphenylcarbonat im Reaktionsgemisch enthalten waren.

#### Beispiel 6:

Katalysatoraktivierung:

In einem Reaktor wurden 0,133 g Palladiumbromid, 0,155 g Kobalt(II)acetylacetonat und 3,2 g Tetrabutylammoniumbromid bei 55°C in 100 g Phenol mit 750 ppm $H_2O$ ($\triangle$ 4 mmol $H_2O$/100 g) gelöst. Durch einen Begasungsrührer wurde eine Stunde Kohlenmonoxid (3 l/h) eingeleitet.

Reaktionsdurchführung:

Zu der Lösung des aktivierten Katalysators wurden 4 g Zeolith A (Baylith L 133 der Fa. Bayer AG) und 0,4 ml 25%ige wäßrige Natronlauge gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 0,8% Diphenylcarbonat im Reaktionsgemisch enthalten waren.

Dieser Versuch wurde, wie beschrieben, wiederholt. Die Analyse ergab, daß 0,8% Diphenylcarbonat im Reaktionsgemisch enthalten waren.

#### Beispiel 7:

In einem Reaktor wurden 0,133 g Palladiumbromid und 3,2 g Tetrabutylammoniumbromid bei 55°C in 100 g Phenol gelöst. Durch einen Begasungsrührer wurde während einer Stunde Kohlenmonoxid (3 l/h) eingeleitet. Zu dieser Lösung wurden 4 g Zeolith A (Baylith L133 der Fa. Bayer AG), 0,153 g Mangan(II)acetylacetonat und 0,302 g Natriumphenolat als Feststoff gegeben. Dann wurde ein Gasgemisch (12 l/h) aus Kohlenmonoxid und Luft (1:1) für 6 Stunden eingeleitet und das Reaktionsgemisch anschließend gaschromatographisch analysiert. Die Analyse ergab, daß 2,5% Diphenylcarbonat im Reaktionsgemisch enthalten waren.

#### Beispiel 7a:

Der Versuch wurde, wie in Beispiel 7 beschrieben, wiederholt, jedoch wurde statt des Natriumphenolats 0,104 mg (2,6 mmol) Natriumhydroxid zugegeben. Die Analyse ergab, daß 0,8% Diphenylcarbonat im Reaktionsgemisch enthalten waren.

#### Beispiel 7b:

Der Versuch wurde, wie in Beispiel 7 beschrieben, wiederholt, jedoch wurde statt des Natriumphenolats 0,4 ml (2,6 mmol) einer 50%igen Natriumhydroxid-Lösung zugegeben. Die Analyse ergab, daß 1,4%

Diphenylcarbonat im Reaktionsgemisch enthalten waren.

#### Beispiel 8:

Der Versuch wurde, wie in Beispiel 7 beschrieben, wiederholt, jedoch wurde das Natriumphenolat nicht als Feststoff, sondern als 15 Gew.-%ige Lösung in Phenol zugegeben. Die Analyse ergab, daß 2,4% Diphenylcarbonat im Reaktionsgemisch enthalten waren.

#### Beispiel 9:

Der Versuch wurde, wie in Beispiel 7 beschrieben, wiederholt, jedoch wurde statt des Natriumphenolats, Kaliumphenolat als Feststoff zugegeben. Die Analyse ergab, daß 2,5% Diphenylcarbonat im Reaktionsgemisch enthalten waren.

#### Beispiel 10:

Der Versuch wurde, wie in Beispiel 7 beschrieben, wiederholt, jedoch wurde statt des Natriumphenolats, Natriumphenolat-trihydrat als Feststoff zugegeben. Die Analyse ergab, daß 2,4% Diphenylcarbonat im Reaktionsgemisch enthalten waren.

#### Beispiel 11:

Der Versuch wurde, wie in Beispiel 3 beschrieben, wiederholt, jedoch wurde statt 25 %iger wäßriger Natronlauge, Natriumphenolat als Feststoff zugesetzt. Die Analyse ergab, daß nach 6 h 2,5 % Diphenylcarbonat im Reaktionsgemisch enthalten waren.

**Patentansprüche**

1. Verfahren zur Herstellung eines organischen Carbonats der Formel (I)

$$R\text{-}O\text{-}CO\text{-}O\text{-}R \qquad (I),$$

in der

R    ein $C_6$-$C_{12}$-Aryl, das ein- bis zweimal durch $C_1$-$C_4$-Alkyl, $C_1$-$C_4$-Alkoxy, Fluor, Chlor oder Brom substituiert sein kann, bedeutet,

durch Umsetzung einer aromatischen Hydroxyverbindung der Formel (II),

$$R\text{-}O\text{-}H \qquad (II),$$

worin

R    die oben angegebene Bedeutung hat,

mit Kohlenmonoxid und Sauerstoff bei 30-200°C, bevorzugt 30-150°C, besonders bevorzugt 40-120°C und bei einem Druck von 1-80 bar, bevorzugt 2-50 bar, besonders bevorzugt 5-25 bar, in Gegenwart einer Verbindung eines Edelmetalls der Gruppe VIIIb als Katalysator, eines Cokatalysators aus der Gruppe der Metallverbindungen der Gruppen III A, IV A, V A, I B, II B, VI B und VII B des Periodensystems der Elemente (Mendelejew), eines quartären Salzes, eines Trockenmittels und einer Base, dadurch gekennzeichnet, daß man den Edelmetallkatalysator vor der Reaktion in Gegenwart des quartären Salzes und in Gegenwart oder in Abwesenheit des Cokatalysators durch Behandlung mit Kohlenmonoxid in flüssiger Phase bei einer Temperatur von 15-200°C, bevorzugt 20-150°C, besonders bevorzugt 40-100°C und einem Druck von 1-300 bar, bevorzugt 1-200 bar, besonders bevorzugt 1-150 bar, aktiviert, wobei der Edelmetall-Katalysator im Aktivierungsansatz in einer Menge von 0,0001-30 Gew.-%, bezogen auf das gesamte Reaktionsgemisch vorliegt.

2. Verfahren nach Anspruch 1, dadurch gekennzeichnet, daß das Edelmetall Palladium ist, bevorzugt in Form von Palladiumbromid.

3. Verfahren nach Ansprüchen 1 und 2, dadurch gekennzeichnet, daß der Cokatalysator im Aktivierungsansatz in einer Menge von 0-40 Gew.-%, bezogen auf den gesamten Aktivierungsansatz, vorliegt.

4. Verfahren nach Ansprüchen 1 bis 3, dadurch gekennzeichnet, daß man als Base ein vorgefertigtes Phenolat oder eine Lösung eines vorgefertigten Phenolats einsetzt.

5. Verfahren nach Anspruch 4, dadurch gekennzeichnet, daß das Phenolat maximal 5 Mol Wasser, bevorzugt maximal 2 Mol, besonders bevorzugt maximal 1 Mol Wasser pro Mol Phenolat enthält und ganz besonders bevorzugt im wesentlichen wasserfrei ist.

6. Verfahren nach Anspruch 4, dadurch gekennzeichnet, daß pro Mol Edelmetall 0,1 bis 500, bevorzugt 0,5 bis 200, besonders bevorzugt 0,9 bis 130 Äquivalente Phenolat eingesetzt werden.

7. Verfahren nach Ansprüchen 1 bis 6, dadurch gekennzeichnet, daß man als quartäres Salz Tetraalkylammonium- oder -phosphoniumsalze in einer Menge von 0,1 bis 50 Gew.-%, bevorzugt 0,5 bis 15 Gew.-%, besonders bevorzugt 1 bis 5 Gew.-%, bezogen auf das Gewicht des Reaktionsgemisches, einsetzt.

8. Verfahren nach den Ansprüchen 1 bis 6, dadurch gekennzeichnet, daß man es ohne Zusatz von Lösungsmitteln durchführt.

9. Verfahren nach den Ansprüchen 1 bis 8, dadurch gekennzeichnet, daß man als Trockenmittel einen Zeolithen, bevorzugt einen des Typs A einsetzt.

10. Verfahren nach den Ansprüchen 1 bis 9, dadurch gekennzeichnet, daß man aus Phenol und CO in Gegenwart von Tetrabutylammoniumbromid, eines Cokatalysators, eines Trockenmittels und eines in Gegenwart eines quartären Salzes mit Kohlenmonoxid aktivierten Palladium-Katalysators sowie eines Alkalimetallphenolats, Diphenylcarbonat herstellt.

**Claims**

1. Process for preparing an organic carbonate of the formula (I)

$$R\text{-}O\text{-}CO\text{-}O\text{-}R \qquad (I),$$

in which

R is a $C_6$-$C_{12}$-aryl which can be monosubstituted or disubstituted by $C_1$-$C_4$-alkyl, $C_1$-$C_4$-alkoxy, flourine, chlorine or bromine,

by reaction of an aromatic hydroxy compound of the formula (II),

$$R\text{-}O\text{-}H \qquad (II),$$

where

R is as defined above,

with carbon monoxide and oxygen at 30-200°C, preferably 30-150°C, particularly preferably 40-120°C, and at a pressure of 1-80 bar, preferably 2-50 bar, particularly preferably 5-25 bar, in the presence of a compound of a noble metal of the group VIIIb as catalyst, a cocatalyst selected from the group consisting of the metal compounds of the groups IIIA, IVA, VA, IB, IIB, VIB and VIIB of the Periodic Table of the Elements (Mendeleev), a quaternary salt, a desiccant and a base, characterized in that the noble metal catalyst is, prior to the reaction, activated by treatment with carbon monoxide in a liquid phase at a temperature of 15-200°C, preferably 20-150°C, particularly preferably 40-100°C and a pressure of 1-300 bar, preferably 1-200 bar, particularly preferably 1-150 bar, in the presence of the quaternary salt and in the presence or in the absence of the cocatalyst, with the noble metal catalyst being present in the activation mixture in an amount of 0.0001-30% by weight, based on the total reaction mixture.

2. Process according to Claim 1, characterized in that the noble metal is palladium, preferably in the form of palladium bromide.

3. Process according to either of Claims 1 and 2, characterized in that the cocatalyst is present in the activation mixture in an amount of 0-40% by weight, based on the total activation mixture.

4. Process according to any of Claims 1 to 3, characterized in that the base used is a preformed phenoxide or a solution of a preformed phenoxide.

5. Process according to Claim 4, characterized in that the phenoxide contains a maximum of 5 mol of water, preferably a maximum of 2 mol, particularly preferably a maximum of 1 mol, of water per mole of phenoxide and is very particularly preferably essentially anhydrous.

6. Process according to Claim 4, characterized in that from 0.1 to 500 equivalents, preferably from 0.5 to 200 equivalents, particularly preferably from 0.9 to 130 equivalents, of phenoxide is used per mole of noble metal.

7. Process according to any of Claims 1 to 6, characterized in that the quaternary salt used is a tetraalkylammonium or tetraalkylphosphonium salt in an amount of from 0.1 to 50% by weight, preferably from 0.5 to 15% by weight, particularly preferably from 1 to 5% by weight, based on the weight of the reaction mixture.

8. Process according to any of Claims 1 to 6, characterized in that it is carried out without addition of solvents.

9. Process according to any of Claims 1 to 8, characterized in that the desiccant used is a zeolite, preferably one of the type A.

10. Process according to any of Claims 1 to 9, characterized in that diphenyl carbonate is prepared from phenol and CO in the presence of tetrabutylammonium bromide, a cocatalyst, a desiccant and a palladium catalyst activated with carbon monoxide in the presence of a quaternary salt, and also an alkali metal phenoxide.

**Revendications**

1. Procédé de préparation d'un carbonate organique de formule (I) :

$$R - O - CO - O - R \qquad (I),$$

où

R     est un alkyle $C_6$-$C_{12}$, qui peut être substitué une ou deux fois par un alkyle $C_1$-$C_4$, un alcoxy $C_1$-$C_4$, un fluor, un chlore ou un brome,

par réaction d'un composé hydroxy aromatique de formule (II) :

$$R\text{-}O\text{-}H \qquad (II),$$

où

R     a la signification donnée ci-dessus,

avec du monoxyde de carbone et de l'oxygène entre 30 et 200°C, de préférence entre 30 et 150°C, surtout entre 40 et 120°C et sous une pression de 1-80 bar, de préférence 2-50 bar, surtout 5-25 bar, en présence d'un composé de métal précieux du groupe VIIIb comme catalyseur, d'un co-catalyseur du groupe des composés métalliques des groupes III A, IV A, V A, I B, II B, VI B et VII B du tableau périodique des éléments (Mendeleiev), d'un sel quaternaire, d'un desséchant et d'une base, caractérisé en ce qu'on active le catalyseur de métal précieux avant la réaction en présence du sel quaternaire et en présence ou en l'absence du co-catalyseur par traitement avec du monoxyde de carbone en phase liquide à une température de 15-200°C, de préférence 20-150°C, surtout entre 40 et 100°C et sous une pression de 1-300 bar, de préférence 1-200 bar, surtout 1-150 bar, où le catalyseur de métal précieux dans le mélange d'activation est présent en une quantité de 0,0001-30 % en poids, par rapport à l'ensemble du mélange réactionnel.

2. Procédé selon la revendication 1, caractérisé en ce que le métal précieux est le palladium, de préférence sous forme de bromure de palladium.

3. Procédé selon les revendications 1 et 2, caractérisé en ce que le co-catalyseur dans le mélange d'activation est présent en une quantité de 0-40 % en poids, par rapport à l'ensemble du mélange d'activation.

4. Procédé selon les revendications 1 à 3, caractérisé en ce qu'on utilise comme base un phénolate préformé ou une solution d'un phénolate préformé.

5. Procédé selon la revendication 4, caractérisé en ce que le phénolate contient au maximum 5 mol d'eau, de préférence au maximum 2 mol, surtout au maximum 1 mol d'eau par mole de phénolate et tout particulièrement on préfère qu'il soit essentiellement anhydre.

6. Procédé selon la revendication 4, caractérisé en ce que par mole de métal précieux, on utilise 0,1 à 500, de préférence 0,5 à 200, surtout 0,9 à 130 éq. de phénolate.

7. Procédé selon les revendications 1 à 6, caractérisé en ce qu'on utilise comme sels quaternaires des sels de tétraalkylammonium ou -phosphonium en une quantité de 0,1 à 50 % en poids, de préférence 0,5 à 15 % en poids, surtout 1 à 5 % en poids, par rapport au poids du mélange réactionnel.

8. Procédé selon les revendications 1 à 6, caractérisé en ce qu'on le réalise sans addition de solvant.

9. Procédé selon les revendications 1 à 8, caractérisé en ce qu'on utilise comme desséchant, une zéolithe, de préférence une zéolithe du type A.

10. Procédé selon les revendications 1 à 9, caractérisé en ce qu'on produit du carbonate de diphényle à partir de phénol et de CO en présence de bromure de tétrabutylammonium, d'un co-catalyseur, d'un desséchant et d'un catalyseur de palladium activé par du monoxyde de carbone en présence d'un sel quaternaire ainsi que d'un phénolate de métal alcalin.